# EUROPÄISCHE PATENTSCHRIFT

(11) **EP 0 448 006 B1**
(45) Veröffentlichungstag und Bekanntmachung des Hinweises auf die Patenterteilung: **25.01.1995**
(21) Anmeldenummer: 91104127.5
(22) Anmeldetag: 18.03.1991
(51) Int. Cl.: C07C 69/14, C07C 67/08

(54) **Verfahren zur Herstellung von Acylalen**
Process for the preparation of acylales
Procédé pour la préparation d'acylales

(30) Priorität: 20.03.1990 DE 4008856
(43) Veröffentlichungstag der Anmeldung: 25.09.1991
(73) Patentinhaber: HOECHST AKTIENGESELLSCHAFT, 65926 Frankfurt am Main (DE)
(72) Erfinder: Hertzsch, Winfried, Dr., W-6000 Frankfurt am Main (DE); Jähne, Gerhard, Dr., W-6230 Frankfurt am Main (DE)

(56) Entgegenhaltungen:
- GB-A- 830 850
- CHEMICAL ABSTRACTS, Band 111, Nr. 7, 14. August 1989, Columbus, Ohio, US; O. ANTONSEN et al.: "Synthesis of sulfinylmethylethers and conversion of these into halomethyl and acyloxymethyl ethers", Seite 707, Zusammenfassung Nr. 57 186e

## Beschreibung

Die vorliegende Erfindung betrifft ein Verfahren zur Herstellung von Acyloxymethoxyalkylverbindungen und Acyloxymethoxyphenylverbindungen (Acylale) 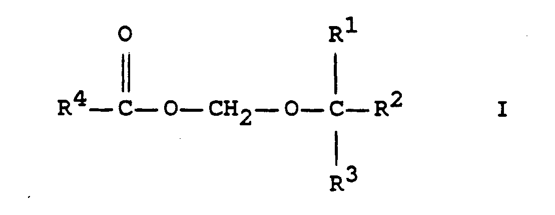
Acetoxymethoxyverbindungen von 2-substituierten Ethandiolen, von 1,3-disubstituierten und von 2,3-disubstituierten 1,2,3-Propantriolen stellen wegen der guten Acetoxyabgangsgruppe wichtige Zwischenprodukte dar.

Die erfindunsgemäß erhältlichen Acylale sind reaktive Verbindungen, die z. B. bei der Synthese von acyclischen Nukleosiden wertvolle Zwischenprodukte darstellen (siehe z.B. C. K. Chu, S. Cutler, J. Heterocyclic Chem., 23, 289 ff (1986)).

Eine lange bekannte Methode zur Herstellung von Acylalen, d. h. von Estern von Halbacetalen besteht in der Umsetzung von äquimolaren Mengen eines Acetals mit einem organischen Säureanhydrid bei erhöhter Temperatur, vorzugsweise in Gegenwart eines sauren Katalysators (siehe z. B. Houben-Weyl, Methoden der organischen Chemie, Georg Thieme Verlag, Stuttgart, 1965, Band VI/3, S.286 f).

Eine weitere Methode bedient sich der Umsetzung von alpha-Halogen-ethern mit Alkalisalzen von Carbonsäuren (siehe z. B. J. W. Farren, J. Am. Chem. Soc. 47, 2422 (1925)).

Eine dritte Methode macht sich die hohe Reaktivität von Vinylethern zunutze und bewerkstelligt die Synthese von Acylalen durch die Addition von organischen Säuren an Vinylether (siehe E. Levas, Comptes Rendues Hebdomadaires des Séances de l'Academie des Sciences, Paris, 228, 1443 (1949)).

Eine kürzlich publizierte Methode schließlich beschreibt die Umsetzung von Sulfinylmethylethern mit Anhydriden organischer Säuren zu Acylalen unter Katalyse mit Methansulfonsäure (siehe O. Antonsen, T. Benneche, K. Undheim, Acta Chem. Scand. B42, 515 ff (1988)).

Die vorliegende Erfindung betrifft ein wesentlich vereinfachtes Verfahren zur Herstellung von Verbindungen der Formel I. Der Vorteil der erfindungsgemäßen Methode gegenüber denen des Standes der Technik liegt darin, daß die Alkoholkomponente des Acylals direkt, d. h. ohne vorherige Isolierung eines aktivierten Zwischenproduktes, eingesetzt werden kann.

Erfindungsgegenstand ist demzufolge ein Verfahren zur Herstellung von Verbindungen der Formel I 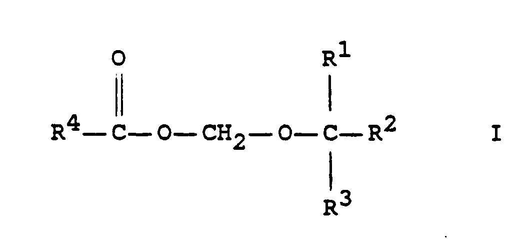
worin
R¹ und/oder R² und/oder R³ Wasserstoff, Phenyl, Alkyl, gegebenenfalls einfach oder mehrfach substituiert mit Alkoxy-, Alkenyloxy-, Alkylthio-, Alkylamino-, Dialkylamino-, Benzyloxy-, Benzylthio-, Benzylamino-, Dibenzylamino-, Acyloxy-, Acylthio-, Acylamino, Diacylamino- und/oder Phthalimidogruppen und/oder mit -P(O)(OR⁵)(OR⁶)-, -P(R⁵)(O)(OR⁶)-, -O-CH₂-P(O)(OR⁵)(OR⁶)- oder -O-CH₂-P(R⁵)(O)(OR⁶)-Resten, wobei R⁵ und R⁶ jeweils unabhängig voneinander Alkyl sein können, bedeuten und
R⁴ Wasserstoff, Alkyl, Trifluormethyl oder Phenyl ist,
oder
R¹ Wasserstoff ist
und
R² und R³ Teil eines verzweigten oder unverzweigten Carbocyclus sind, wobei der Carbocyclus ein oder mehrere Hydroxy-, Mercapto-, Amino- oder Alkylamino-Gruppen enthalten kann, welche mit Alkyl- und/oder Acyl- und/oder Benzylgruppen blockiert sind
und
R⁴ wie oben definiert ist,
oder
R¹ für eine zusätzliche Bindung zwischen dem tertiären Kohlenstoffatom und R² oder R³ steht
und
R² und R³ Teil eines Phenylringes sind, der noch weiter mit Alkylgruppen und/oder Hydroxy-, Mercapto-, Amino- oder Alkylamino-Gruppen substituiert sein kann, welche mit Acyl- und/oder Benzyl- und/oder Alkyl-Gruppen blockiert sind
und
R⁴ wie oben definiert ist,
dadurch gekennzeichnet, daß eine Verbindung der Formel 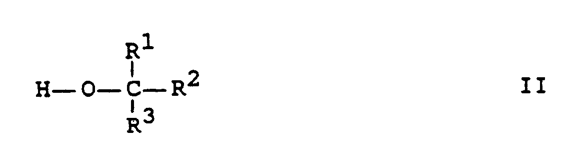
mit einer Verbindung der Formel III, 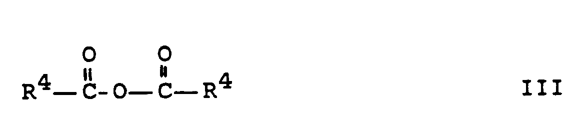
in Gegenwart von Dimethylsulfoxid und der organischen Säure, deren Anhydrid der Formel III Verwendung findet, umgesetzt wird,
wobei die Reste R¹ - R⁴ die obengenannten Bedeutungen haben
Besonders geeignet ist das Verfahren zur Herstellung von Verbindungen der Formel I in denen
R¹ und/oder R² und/oder R³ Wasserstoff, Phenyl, C₁-C₁₂-Alkyl, gegebenenfalls bis zu dreifach substituiert mit C₁-C₆-Alkoxy-, C₁-C₆-Alkylthio-, C₁-C₆-Alkylamino, C₁-C₁₂-Dialkylamino-, Benzyloxy-, Benzylthio, Benzylamino-, Dibenzylamino-, C₁-C₈-Acyloxy-, C₁-C₈-Acylthio, C₁-C₈-Acylamino- und/oder C₂-C₁₆-Diacylaminogruppen, wobei die Acylgruppen aliphatisch - beispielsweise Acetoxy oder Pivaloyloxy - oder aromatisch - beispielsweise Benzoyloxy sein können und/oder mit -P(O)(OR⁵)(OR⁶)-, -P(R⁵)(O)(OR⁶)-, -O-CH₂-P(O)(OR⁵)(OR⁶)- oder -O-CH₂-P(R⁵)(O)(OR⁶)-Resten, wobei R⁵ und R⁶ jeweils unabhängig voneinander C₁-C₆-Alkyl sein können, bedeuten und
R⁴ Wasserstoff, Alkyl, Trifluormethyl oder Phenyl bedeutet
oder
R¹ Wasserstoff ist
und
R² und R³ Teil eines verzweigten oder unverzweigten Carbocyclus mit 2 bis 8 Kohlenstoffatomen, vorzugsweise 3 bis 5 Kohlenstoffatomen im Carbocyclus sind, wobei der Carbocyclus ein oder mehrere Hydroxy-, Mercapto-, Amino- oder C₁-C₆-Alkylamino-Gruppen enthalten kann, welche mit C₁-C₆ Alkyl- und/oder C₁-C₈ Acyl- und/oder Benzylgruppen blockiert sind
und
R⁴ wie oben definiert ist,
oder
R¹ für eine zusätzliche Bindung zwischen dem tertiären Kohlenstoffatom und R² oder R³ steht
und
R² und R³ Teil eines Phenylringes sind, der noch weiter mit C₁-C₄ Alkylgruppen und/oder Hydroxy-, Mercapto-, Amino- oder C₁-C₄ Alkylamino-Gruppen substituiert sein kann, welche mit C₁-C₈ Acyl und/oder Benzyl- und/oder C₁-C₆ Alkyl-Gruppen blockiert sind
und
R⁴ wie oben definiert ist.

Besonders bevorzugt ist das Verfahren zur Herstellung von Verbindungen der Formel I, in denen
R¹ und R² Wasserstoff und R³ C₁-C₁₂-Alkyl oder R¹ Wasserstoff und R² und R³ C₁-C₁₂-Alkyl sind, wobei dieses C₁-C₁₂-Alkyl bis zu zweifach mit C₁-C₆-Alkoxy-, C₁-C₆-Alkylthio-, Benzyloxy-, Benzylthio-, Dibenzylamino-, C₁-C₈-Acyloxy-, C₁-C₈-Acylthio- und/oder C₂-C₁₆-Diacylaminogruppen - wobei die Acylgruppen aliphatisch - beispielsweise Acetoxy oder Pivaloyloxy - oder aromatisch - beispielsweise Benzoyloxy - sein können - und/oder mit Resten -P(O)(OR⁵)(OR⁶), -P(R⁵)(O)(OR⁶), -O-CH₂-P-(O)(OR⁵)(OR⁶) oder -O-CH₂-P(R⁵)(O)(OR⁶), wobei R⁵ und R⁶ jeweils unabhängig voneinander C₁-C₆-Alkyl bedeuten, substituiert sein kann und R⁴ Alkyl, Trifluormethyl oder Phenyl bedeutet.

Das obengenannte Verfahren eignet sich besonders zur Herstellung der oben genannten Verbindungen, in denen R⁴ C₁-C₆-Alkyl, insbesondere C₁-C₃-Alkyl ist.

Eine ganz besondere Bedeutung besitzt das erfindungsgemäße Verfahren für die Umsetzung von Acetanhydrid als Verbindung der Formel III (d.h. R⁴ = CH₃).

Wenn die Reste R¹ und/oder R² und/oder R³ der obengenannten Verbindungen Acylgruppen tragen, erhält man besonders einheitliche Produkte, wenn diese Acylgruppen mit der R⁴-C(O)-Gruppe in Formel III identisch sind.

Die als Substituenten genannten Alkylgruppen können geradkettig, verzweigt oder cyclisch sein.

Die genannten Acylgruppen können geradkettig, verzweigt, cycloaliphatisch oder aromatisch sein.

Die Verbindungen der Formel I können ein oder mehrere chirale Zentren aufweisen. Die Verbindungen liegen in der Regel als Racemate vor; eine Herstellung bzw. Isolierung der reinen Enantiomere ist möglich. Gegenstand der Erfindung ist deshalb sowohl das Verfahren zur Herstellung der reinen Enantiomeren als auch Mischungen derselben, wie z. B. das zugehörige Racemat.

Die Alkyl-, Acyl- oder Benzyl-Schutzgruppen, mit denen Hydroxy-, Mercapto- oder Aminogruppen der Substituenten R¹ bis R³ gegebenenfalls blockiert sind, können nach üblichen Methoden abgespalten werden, wie z.B. durch Reaktion mit Borhalogeniden, Hydrolyse oder Hydrogenolyse.

Zur Durchführung des erfindungsgemäßen Verfahrens werden vorzugsweise ein Alkanol der Formel II, ein Anhydrid der Formel III, die dem Anhydrid entsprechende Carbonsäure und trockenes Dimethylsulfoxid unter Rühren bei einer Temperatur von -10 bis 40 °C, besonders bevorzugt von 0 bis 30 °C zusammengegeben. Das Molverhältnis von Anhydrid: Carbonsäure:Dimethylsulfoxid zu 1 Mol Alkanol liegt vorzugsweise bei 2-10:1,5-20:1-30, besonders bevorzugt bei 4-6:6-14:10-20, insbesondere bei ca. 5:ca.10:ca.15. Das Reaktionsgemisch wird vorzugsweise 1-24 Stunden, besonders bevorzugt 5 bis 7 Stunden bei 0-100 °C, insbesondere bei 30-90 °C gerührt.

Das Reaktionsgemisch kann nach herkömmlichen Methoden aufgearbeitet werden.
Vorzugsweise erfolgt die Aufarbeitung z.B. derart, daß das erhaltene Reaktionsgemisch in Eiswasser gegossen und dreimal mit einem Ether, vorzugsweise Diethylether oder Diisopropylether ausgeschüttelt wird. Die organische Phase wird danach 3 x mit Wasser und dann mehrere Male mit konzentrierter Natriumhydrogencarbonatlösung ausgeschüttelt bis die wäßrigen Waschwasser nicht mehr sauer reagieren. Sodann wird noch einmal mit Wasser ausgeschüttelt und die organische Phase über Natriumsulfat getrocknet, filtriert und unter vermindertem Druck vom Lösungsmittel befreit. Der Rückstand wird z.B. einer fraktionierten Destillation unterworfen und liefert neben Acyloxymethylthiomethylether die Acyloxymethylverbindung des eingesetzten Alkanols.

Die vorliegende Erfindung wird durch das nachfolgende Ausführungsbeispiel näher erläutert.

### Beispiel:

Verbindung der Formel I, worin R1 = Wasserstoff, R2 = R3 = Isopropoxymethyl, R4 = Methyl ist:
Zu einer Mischung aus 120 ml Eisessig und 100 ml Essigsäureanhydrid werden unter Rühren 200 ml trockenes Dimethylsulfoxid so zugetropft, daß die Temperatur des Gemisches nicht über 35 Grad Celsius ansteigt. Man rührt weitere 30 Minuten, bevor 35.2 g (0.2 Mol) 1,3-Bis-isopropoxy-propan-2-ol (hergestellt durch Reaktion von Natriumisopropylat mit 2,3-Epoxypropyl-isopropylether in Isopropanol) zugetropft werden. Nach Beendigung der Zugabe wird die Mischung 7 Stunden lang auf 90 - 100 Grad Celsius erhitzt. Die abgekühlte Reaktionsmischung wird in Eiswasser gegossen und mehrmals mit Diethylether ausgeschüttelt. Die organische Phase wird dann mit Wasser und anschließend mit konzentrierter wäßriger Natriumhydrogencarbonatlösung gewaschen, über Natriumsulfat getrocknet und unter vermindertem Druck eingeengt. Es hinterbleibt ein hellgelbes Öl, das einer fraktionierten Destillation unterworfen wird. Ein Vorlauf mit Siedepunkt 46 - 47 Grad Celsius bei einem Druck von 2 kPa (15 mm Hg) besteht aus Acetoxymethylthiomethylether. Das Reaktionsprodukt, 2-Acetoxymethoxy-1,3-bis-isopropoxy-propan, siedet bei 87 - 92 Grad Celsius bei einem Druck von 133 Pa (1 mm Hg). Die Ausbeute beträgt 27.3 g (55% d. Th.).
1H-NMR (60 MHz, CDCl₃), δ [ppm]: 5.43 (s, 2H), 4.0 - 3.33 (m, 7H), 2.12 (s, 3H), 1.33 (d, 12H).

In ähnlicher Weise können hergestellt werden:
1-Acetoxymethoxy-2-methoxy-ethan
1-Acetoxymethoxy-2-ethoxy-ethan
1-Acetoxymethoxy-2-propoxy-ethan
1-Acetoxymethoxy-2-isopropoxy-ethan
1-Trifluoracetoxymethoxy-2-isopropoxy-ethan
1-Benzoyloxymethoxy-2-isopropoxy-ethan
1-Acetoxymethoxy-2-butoxy-ethan
1-Acetoxymethoxy-2-benzyloxy-ethan
1-Acetoxymethoxy-2-phenoxy-ethan
1-Acetoxymethoxy-2-acetoxy-ethan
1-Acetoxymethoxy-2-pivaloyloxy-ethan
2-Acetoxymethoxy-3-methoxy-propan
(2-Acetoxymethoxy-ethoxy)methan-phosphonsäure-di-isopropylester
((2-Acetoxymethoxy-ethoxy)methyl)-methyl-phosphinsäure-isopropylester
(3-Acetoxymethoxy)propan-phosphonsäure-di-isopropylester
1-Acetoxymethoxy-2-benzyloxy-3-isopropoxy-propan
1-Benzoyloxymethoxy-2-benzyloxy-3-isopropoxy-propan
1-Acetoxymethoxy-2-benzyloxy-3-methoxy-propan
1-Acetoxymethoxy-2-benzyloxy-3-ethoxy-propan
1-Acetoxymethoxy-2,3-bis-benzyloxy-propan
1-Acetoxymethoxy-2,3-bis-(isopropoxy)-propan
1-Acetoxymethoxy-2-benzyloxy-3-(N-phthalimido)-propan
1-Acetoxymethoxy-2-benzyloxy-3-methylthio-propan
1-Acetoxymethoxy-2-benzyloxy-3-ethylthio-propan
(4-Acetoxymethoxy-3-benzyloxy)butan-phosphonsäure-di-isopropylester
2-Acetoxymethoxy-1,3-bis(methoxy)-propan
2-Acetoxymethoxy-1,3-bis(ethoxy)-propan
2-Acetoxymethoxy-1,3-bis(propoxy)-propan
2-Acetoxymethoxy-1,3-bis(isopropoxy)-propan
2-Trifluoracetoxymethoxy-1,3-bis(isopropoxy)-propan
2-Benzoyloxymethoxy-1,3-bis(isopropoxy)-propan
2-Acetoxymethoxy-1,3-bis(prop-2-en-1-oxy)-propan
2-Acetoxymethoxy-1,3-bis(benzyloxy)-propan
2-Acetoxymethoxy-1-isopropoxy-3-(1,1,2,2-tetrafluorethoxy)-propan
(3-Acetoxymethoxy-4-benzyloxy)butan-phosphonsäure-di-isopropylester
2-Acetoxymethoxy-1,3-bis(cyclopentyloxy)-propan
2-Acetoxymethoxy-1,3-bis(cyclohexyloxy)-propan
2-Acetoxymethoxy-1-benzyloxy-3-isopropoxy-propan
2-Acetoxymethoxy-1-benzyloxy-3-methoxy-propan
2-Acetoxymethoxy-1-isopropoxy-3-methoxy-propan
2-Acetoxymethoxy-1-isopropoxy-3-[1,3-bis(isopropoxy)-2-propoxy]-propan
2-Acetoxymethoxy-1-isopropoxy-3-pivaloyloxy-propan
2-Acetoxymethoxy-1-benzyloxy-3-pivaloyloxy-propan
2-Acetoxymethoxy-1,3-bis(pivaloyloxy)-propan
2-Acetoxymethoxy-1-isopropoxy-3-phenoxy-propan
2-Acetoxymethoxy-1-isopropoxy-3-(N-phthalimido)-propan
Acetoxymethoxy-cyclohexan
Acetoxymethoxy-benzol
In ähnlicher Weise können unter Verwendung von Propionsäure und Propionsäureanhydrid oder von Buttersäure und Butteräureanhydrid oder von Isobuttersäure und Isobuttersäureanhydrid die entsprechenden Propanoyloxymethoxy- oder Butanoyloxymethoxy- oder Isobutanoyloxymethoxyverbindungen gewonnen werden.

## Patentansprüche

1. Verfahren zur Herstellung von Verbindungen der Formel I, 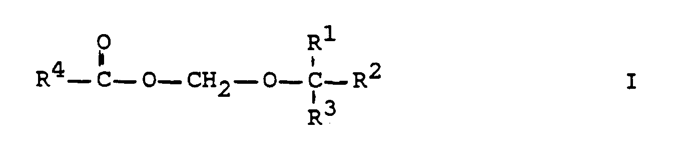 worin
R¹ und/oder R² und/oder R³ Wasserstoff, Phenyl, Alkyl, gegebenenfalls einfach oder mehrfach substituiert mit Alkoxy-, Alkenyloxy-, Alkylthio-, Alkylamino-, Dialkylamino-, Benzyloxy-, Benzylthio-, Benzylamino-, Dibenzylamino-, Acyloxy-, Acylthio-, Acylamino-, Diacylamino- und/oder Phthalimidogruppen und/oder mit -P(O)(OR⁵)(OR⁶)-, -P(R⁵)(O)(OR⁶)-, -O-CH₂-P(O)(OR⁵)(OR⁶)- oder -O-CH₂-P(R⁵)(O)(OR⁶)-Resten, wobei R⁵ und R⁶ jeweils unabhängig voneinander Alkyl sein können, bedeuten und
R⁴ Wasserstoff, Alkyl, Trifluormethyl oder Phenyl ist, oder
R¹ Wasserstoff ist
und
R² und R³ Teil eines verzweigten oder unverzweigten Carbocyclus sind, wobei der Carbocyclus ein oder mehrere Hydroxy-, Mercapto-, Amino- oder Alkylamino-Gruppen enthalten kann, welche mit Alkyl- und/oder Acyl- und/oder Benzylgruppen blockiert sind
und
R⁴ wie oben definiert ist,
oder
R¹ für eine zusätzliche Bindung zwischen dem tertiären Kohlenstoffatom und R² oder R³ steht
und
R² und R³ Teil eines Phenylringes sind, der noch weiter mit Alkylgruppen und/oder Hydroxy-, Mercapto-, Amino- oder Alkylamino-Gruppen substituiert sein kann, welche mit Acyl- und/oder Benzyl- und/oder Alkyl-Gruppen blockiert sind
und
R⁴ wie oben definiert ist,
dadurch gekennzeichnet, daß eine Verbindung der Formel II 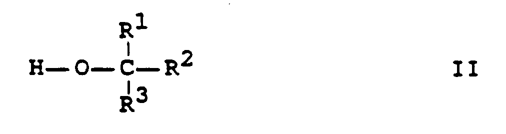 mit einer Verbindung der Formel III, 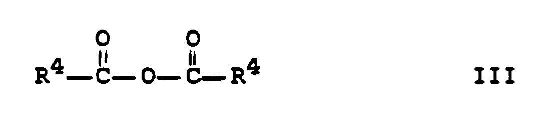 in Gegenwart von Dimethylsulfoxid und der organischen Säure, deren Anhydrid der Formel III Verwendung findet, umgesetzt wird,
wobei die Reste R¹- R⁴ die obengenannten Bedeutungen haben.

2. Verfahren gemäß Anspruch 1, dadurch gekennzeichnet, daß das Molverhältnis der einzusetzenden Reaktanden Anhydrid der Formel III/Carbonsäure/Dimethylsulfoxid pro Mol Alkanol der Formel II bei 2-10/1,5-20/1-30 liegt.

3. Verfahren gemäß den Ansprüchen 1 oder 2, dadurch gekennzeichnet, daß die Reaktanden bei einer Temperatur von -10 - 30 °C unter Rühren zusammengegeben werden und das erhaltene Reaktionsgemisch 1 bis 24 Stunden bei einer Temperatur von 0 bis 100 °C gerührt wird.

## Claims

1. A process for the preparation of a compound of the formula I 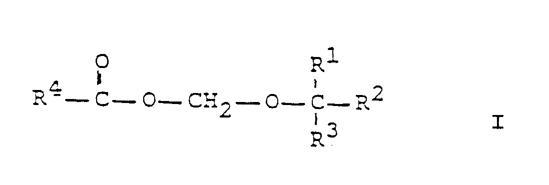 in which
R¹ and/or R² and/or R³ are hydrogen, phenyl or alkyl which is optionally substituted by one or more alkoxy, alkenyloxy, alkylthio, alkylamino, dialkylamino, benzyloxy, benzylthio, benzylamino, dibenzylamino, acyloxy, acylthio, acylamino, diacylamino and/or phthalimido groups and/or by -P(O)(OR⁵)(OR⁶), -P(R⁵)(O)(OR⁶), -O-CH₂-P(O)(OR⁵)(OR⁶) or -O-CH₂-P(R⁵)(O)(OR⁶) radicals, in which R⁵ and R⁶ in each case independently of one another can be alkyl, and
R⁴ is hydrogen, alkyl, trifluoromethyl or phenyl,
or
R¹ is hydrogen
and
R² and R³ are part of a branched or unbranched carbocyclic radical, it being possible for the carbocyclic radical to contain one or more hydroxyl, mercapto, amino or alkylamino groups which are blocked by alkyl and/or acyl and/or benzyl groups,
and
R⁴ is as defined above,
or
R¹ is an additional bond between the tertiary carbon atom and R² or R³
and
R² and R³ are part of a phenyl ring, which can also be further substituted by alkyl groups and/or hydroxyl, mercapto, amino or alkylamino groups which are blocked by acyl and/or benzyl and/or alkyl groups,
and
R⁴ is as defined above,
which comprises reacting a compound of the formula II 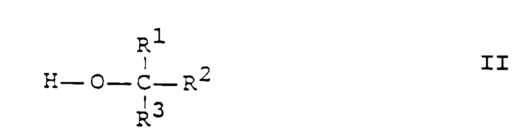 with a compound of the formula III 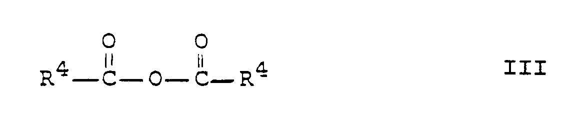 in the presence of dimethyl sulfoxide and of the organic acid of which the anhydride of the formula III is used, in which the radicals R¹ - R⁴ have the abovementioned meanings.

2. The process as claimed in claim 1, wherein the molar ratio of the reactants anhydride of the formula III/carboxylic acid/dimethyl sulfoxide to be employed per mol of alkanol of the formula II is 2-10/1.5-20/1-30.

3. The process as claimed in claim 1 or 2, wherein the reactants are brought together at a temperature of -10 - 30°C, while stirring, and the resulting reaction mixture is stirred at a temperature of 0 to 100°C for 1 to 24 hours.

## Revendications

1. Procédé pour la préparation de composés de formule I 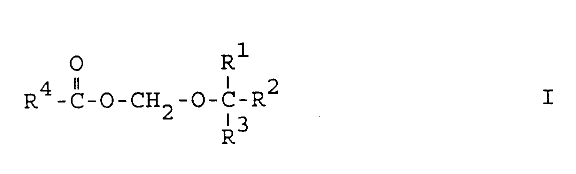 dans laquelle
R¹ et/ou R² et/ou R³ représentent un atome d'hydrogène ou un groupe phényle ou alkyle éventuellement une ou plusieurs fois substitué par un ou des radicaux alcoxy, alcényloxy, alkylthio, alkylamino, dialkylamino, benzyloxy, benzylthio, benzylamino, dibenzylamino, acyloxy, acylthio, acylamino, diacylamino et/ou phtalimido et/ou par des radicaux -P(O)(OR⁵)(OR⁶), -P(R⁵)(O)(OR⁶), -O-CH₂-P(O)(OR⁵)(OR⁶) ou -O-CH₂-P(R⁵)(O)(OR⁶), R⁵ et R⁶ pouvant être chacun, indépendamment l'un de l'autre, un groupe alkyle, et
R⁴ est un atome d'hydrogène ou un groupe alkyle, trifluorométhyle ou phényle,
ou
R¹ est un atome d'hydrogène,
et
R² et R³ font partie d'un cycle carbocyclique ramifié ou non ramifié, le cycle carbocyclique pouvant contenir un ou plusieurs groupes hydroxy, mercapto, amino ou alkylamino, qui sont bloqués par des groupes alkyle et/ou acyle et/ou benzyle,
et
R⁴ est tel que défini ci-dessus,
ou
R¹ représente une liaison supplémentaire entre l'atome de carbone tertiaire et R² ou R³,
et
R² et R³ font partie d'un cycle phényle qui peut être substitué davantage par des groupes alkyle et/ou hydroxy, mercapto, amino ou alkylamino, qui sont bloqués par des groupes acyle et/ou benzyle et/ou alkyle,
et
R⁴ est tel que défini plus haut,
caractérisé en ce que l'on fait réagir un composé de formule II 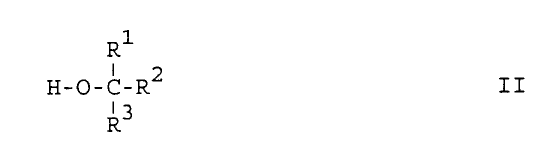 avec un composé de formule III 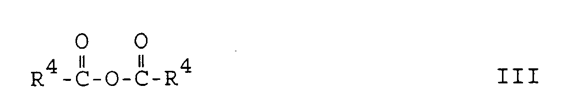 en présence de diméthylsulfoxyde et de l'acide organique dont on utilise l'anhydride de formule III,
les radicaux R¹-R⁴ ayant les significations données plus haut.

2. Procédé selon la revendication 1, caractérisé en ce que le rapport molaire des partenaires réactionnels anhydride de formule III/acide carboxylique/diméthylsulfoxyde à utiliser, par mole d'alcanol de formule II, est de 2-10:1,5-20:1-30.

3. Procédé selon la revendication 1 ou 2, caractérisé en ce que les partenaires réactionnels sont mis en contact à une température de -10 à +30°C, sous agitation, et le mélange réactionnel obtenu est agité pendant 1 à 24 heures à une température de 0 à 100°C.
